# EUROPEAN PATENT SPECIFICATION

(11) **EP 0 847 280 B1**
(45) Date of publication and mention of the grant of the patent: **20.11.2002**
(21) Application number: 96928566.7
(22) Date of filing: 23.08.1996
(51) Int. Cl.: A61K 47/48, A61P 37/00, A61P 37/06

(54) **COMPOSITIONS AND THEIR USES AGAINST HYPERACUTE REJECTION OF XENOGRAFTS AND CERTAIN DISEASES**
ZUSAMMENSETZUNGEN UND IHRE ANWENDUNGEN GEGEN DIE HYPERAKUTE ABSTOSSUNG VON TRANSPLANTATEN UND BESTIMMTE KRANKHEITEN
COMPOSITIONS ET LEURS UTILISATIONS CONTRE LE REJET HYPERAIGUE DE XENOGREFFES ET CONTRE CERTAINES MALADIES

(30) Priority: 31.08.1995 GB 9517758
(43) Date of publication of application: 17.06.1998
(73) Proprietor: Imutran Limited, Cambridge CB2 2AH (GB)
(72) Inventor: WARNER, Richard, Gareth, London EC1A 2DY (GB)
(74) Representative: Boos, Harald
(86) International application number: GB9602078
(87) International publication number: WO97007823

(56) References cited:
- EP-A- 0 180 490
- EP-A- 0 417 927
- WO-A-96/03144
- US-A- 5 401 723
- J. EXP. MED. (1987), 166(2), 419-32 CODEN: JEMEAV;ISSN: 0022-1007, 1987, XP002027056 TOWBIN, HARRY ET AL: "Circulating antibodies to mouse laminin in Chagas disease, American cutaneous leishmaniasis, and normal individuals recognize terminal galactosyl(.alpha.1-3)-galactose epitopes"
- CHEMICAL ABSTRACTS, vol. 101, no. 1, 2 July 1984 Columbus, Ohio, US; abstract no. 5268, EKWALL, ERIK ET AL: "Specific identification of Salmonella serogroup E antigen O3 by immunofluorescence and coagglutination with antiserum elicited by a synthetic trisaccharide-bovine serum albumin glycoconjugate" XP002027058 & J. CLIN. MICROBIOL. (1984), 19(5), 699-702 CODEN: JCMIDW;ISSN: 0095-1137, 1984,
- J. IMMUNOL. (1995), 154(10), 5500-10 CODEN: JOIMA3;ISSN: 0022-1767, 1995, XP002027057 COLLINS, BRADLEY H. ET AL: "Cardiac xenografts between primate species provide evidence for the importance of the.alpha.-galactosyl determinant in hyperacute rejection"
- DATABASE BIOSIS BIOSCIENCES INFORMATION SERVICE, PHILADELPHIA, PA, US AN=96:406292, XP002027059 cited in the application & XENOTRANSPLANTATION, vol. 2, no. 2, 1995, pages 95-106, RIEBEN R. ET AL.: "DETECTION, IMMUNOABSORPTION AND INHIBITION OF CYTOTOXIC ACTIVITY OF ANTI-ALPHA-GAL ANTIBODIES USING NEWLY DEVEVLOPED SUBSTANCES WITH SYNTHETIC GAL ALPHA-1-3GAL DISACCHARIDE EPITOPES."

## Description

The present invention relates to the field of transplantation and, in particular, to preventing hyperacute rejection of xenografts or at least reducing the extent or rate of rejection.

Transplantation of a pig organ (e.g. heart, kidney) into a primate triggers a rapid (sub-10 minute) hyperacute rejection in the recipient which destroys the donor tissue.

The first step in hyperacute rejection is considered to be the binding of pre-formed "xenogenic natural antibodies" (XNA) of the recipient to the blood vessels of the donor tissue which in turn activates the complement system-of the recipient. There is a strong evidence that the major target of these antibodies is galactose al,3galactose (Galα1,3Gal) (Sandrin et *al, Proc Natl Acad Sci USA* **90:** 11391 (1993)). This is a carbohydrate which is widely expressed on pig tissue, but absent from humans (Galili *et al*, *Proc Natl Acad Sci USA* **84**: 1369-1373 (1987)). This disaccharide is a terminal modification of the oligosaccharide chains displayed by cell-surface glycoproteins and glycolipids. A number of porcine endothelial proteins which bind XNA, and therefore probably carry Galal,3Gal, have been identified (Platt *et al*, *Transplantation* **57*:*** 327-335 (1994)).

The majority of XNAs directed against porcine tissues are known to bind specifically Galα1,3Gal. Oligosaccharides containing this structure (B-trisaccharide: Galα1,3Galβ1,4GlcNAc; B-disaccharide: Galα1,3Gal) are the best inhibitors of the cytotoxic effects of human serum on porcine endothelial cells (Neethling *et al, Transplantation* **57:** 959-963 (1994)). Other α-galactosylated sugars (not 1,3-linked) do inhibit, but far less efficiently.

Cooper and co-workers have examined how infusion of the α-galactosylated sugars arabinogalactan and melibiose into baboons affects the ability of serum from these animals to kill pig endothelial cells *in vitro* (Ye *et al*, *Transplantation* **58**: 330-337 (1994)). A level of protection was afforded, but not sufficient to significantly extend the life of a heterotropic pig heart xenotransplant. In addition, high levels of carbohydrate infusion can make the animals ill (diuresis, respiratory distress).

The group of Cooper *et al* have also attempted to inhibit antibody-mediated lysis of porcine cells *in vitro* with B-disaccharide-polyacrylamide(PAA) conjugates commercially available from Syntesome GmbH, Fine Biochemicals, Heimdall Str. 4, D-81739 Munich, Germany. (Riebin *et al*, *Xenotransplantation* **2**: 98-106 (1995)). The disaccharide can be attached to polyacrylamide at a range of substitution densities, which vary in their ability to protect pig tissue from attack. However, no direct comparisons of the protective capability of the PAA conjugate and free disaccharide have been published.

WO 93/03735 discloses various methods and compositions which are said to be useful for attenuating antibody-mediated xenograft rejections. In one embodiment, xenoantigens are coupled to solid supports and are indicated as being useful for perfusion of a patient's blood in order to remove xenoantibodies. Examples of suitable solid supports are said to be silica, synthetic silicates such as porous glass, biogenic silicates such as diatomaceous earth, silicate containing minerals such as kaolinite, and synthetic polymers such as polystyrene, polypropylene and polysaccharides.

According to the present invention there is provided a synthetic conjugate of a protein and a plurality of epitopes, for use in medicine; wherein said epitopes are capable of being bound by xenogenic natural antibodies.

The term "protein" is used herein to include moieties with peptide bonds, such as peptides (e.g. polylysine), polypeptides and complete proteins. It should therefore not be construed in an unduly limiting manner. If desired, the characteristics (e.g. the charge characteristics) of a protein for use in the present invention may be modified. For example, methoxyethoxyacetylation of the epsilon-amino group of lysine in poly (L-lysine) reduces non-specific interactions between it and tissues as a result of the polypeptide's cationic charges (Gasho, A., *et al*, *Biol. Pharm. Bull.* **17**:275-282 (1994)).

The term "synthetic" is used here to indicate that the conjugate does not occur naturally.

The term "conjugate" indicates that the protein and epitopes are linked together.

The term "xenogenic natural antibody" is used here to include both:
(a) antibodies which are already present in a host and which can participate in rejection of xenografts in that host. (These antibodies are thought to be involved in hyperacute rejection of xenografts.)
and (b) antibodies which are newly synthesised in a host in response to the present of a xenograft in that host. (These are sometimes referred to as "xenoreactive elicited antibodies" and are thought to be involved in acute rejection of xenografts).

The present inventors have discovered that a conjugate according to the present invention is highly effective in binding to xenogenic natural antibodies and is particularly effective in binding IgM. This finding is significant since IgM is implicated in the early stages of rejection and is generally believed to be the most important immunoglobulin class in this process. Blocking of IgM binding to epitopes expressed on a xenograft is therefore a significant advance in preventing or alleviating rejection.

Since a large number of epitopes can be bound to a single protein molecule the total number of molecules required to present a given number of epitopes to a patient's immune system can be significantly reduced relative to the use of individual epitopes. The conjugate can be used to avoid osmotic disturbances or to result in fewer osmotic disturbances in a patient (in comparison to the free sugar) since osmolarity is directly related to the concentration of molecules in solution.

Preferably the epitopes will bind to human xenogenic natural antibodies involved in the rejection (e.g. acute or hyperacute rejection) of xenografts taken from pigs.

However, the epitopes may bind to human xenogenic natural antibodies involved in the rejection of xenografts taken from other animals. Preferred epitopes are therefore not normally present in the human adult. Particular epitopes which can be used are discussed later on.

The present invention makes available a method of preventing rejection of a xenograft, or at least of reducing the extent or rate of rejection, by administering the conjugate to a recipient of the xenograft. (This may be done before, during and/or after implanting the xenograft. Preferably it is done before implantation, e.g. up to twenty-four hours before a transplant operation. Booster doses may then be administered subsequently). The conjugate can bind to xenogenic natural antibodies present in the recipient and can therefore block the binding of these antibodies to epitopes present on the xenograft. This blocking effect removes many free xenogenic natural antibodies from the circulation of a recipient and these antibodies are therefore not free to activate complement by binding to epitopes present on the xenograft. Complement activation is believed to be a major factor in hyperacute rejection of xenografts and the blocking effect which can be achieved using the conjugate of the present invention is therefore of major significance. Indeed even if this blocking effect is only short-lived (e.g. if it lasts only for a few days) then there are indications in the art that it can still be effective since accommodation of a xenograft may occur. This is discussed by Rieben *et al* (*supra*), where an analogy is drawn with the ABO system. In any event it is possible to monitor a recipient and to provide additional conjugate of the present invention when necessary.

Conjugates of the present invention can function as tolerogens i.e. as molecules which can reduce immunogenicity in response to subsequent challenge with a hapten. Thus administration of conjugates of the present invention prior to inserting the pig xenograft may inhibit an antibody response to the graft itself, through tolerance. When a graft is already in place, the conjugate may be administered to prevent an antibody response to the graft by inhibition (i.e. by reducing the production of anti-hapten antibody).

(By way of background, the work of Dintzis (e.g. Dintzis, R.Z., *et al*, *J*. *Immunol.* **131**:2196-2203 (1983)) with multivalent polyacrylamide conjugates can be referred to. This has demonstrated that conjugates presenting multiple haptenic groups can be either immunogenic (that is, will stimulate anti-hapten antibody production) or inhibitory (reduce the production of anti-hapten antibody in the presence of immunogenic conjugates). Whether the conjugate is immunogenic or inhibitory depends on the conjugate size and hapten density. If administration of an inhibitory conjugate before challenge with an immunogenic conjugate reduces the immunogenicity of the latter, the inhibitory molecule is termed tolerogenic. All these effects (immunogenicity, inhibition, and tolerogenicity) are thought to operate through competition at the hapten receptors on B-cells. Tolerogenic inhibitory molecules are envisaged as persisting at the B-cell receptor in spite of removal from the surrounding environment.)

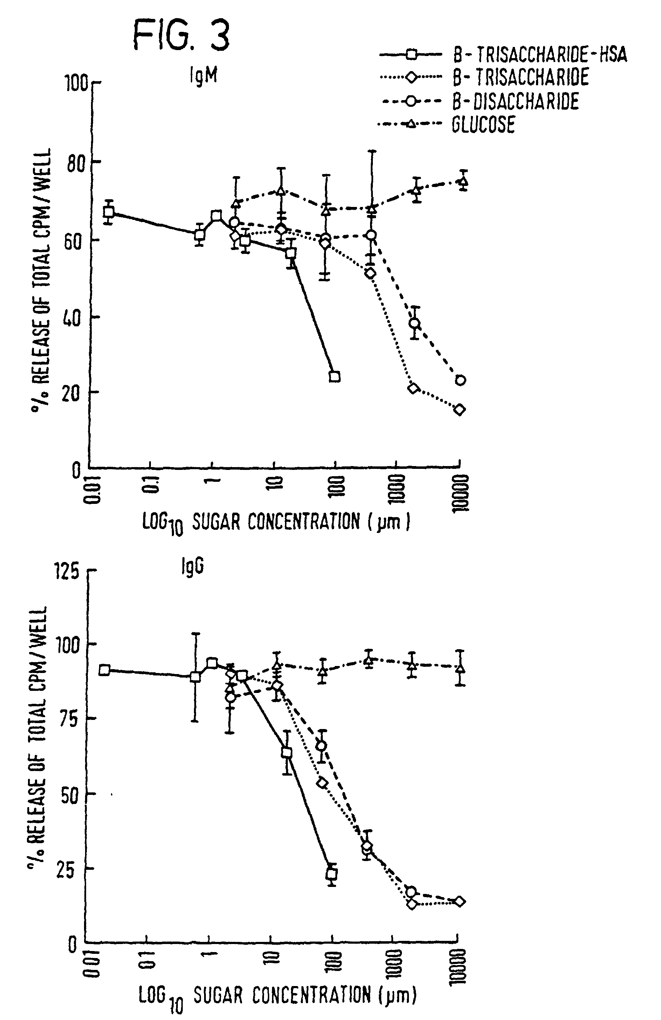

The conjugates used in the present invention can be administered to a patient by any appropriate technique, but is preferably administered by infusion (by means of a drip). Dosage levels can be determined by those skilled in the art and will depend upon factors such as the mass of the patient, the size of the xenograft, the size of the protein present in the conjugate, the number of epitopes present in the conjugate, the spacing of such epitopes, etc. Without being bound by theory, a typical dosage for the B-trisaccharide-HSA referred to in the Example might be 0.1mg/ml to 5mg/ml final concentration in the serum. This range is derived from the concentration of conjugate observed to fully inhibit the lysis of endothelial cells *in vitro* by 10µg/ml XNA IgM (see Figure 3, which will be described later), which is approximately 0.5mg/ml. 10µg/ml represents the approximate concentration of XNA IgM expected in the serum. Dosage ranges for other conjugates of the present invention can be approximated using a similar assay procedure. Animal experiments may then be used to determine dosage levels more accurately.

As indicated above, a patient can be monitored and additional doses of the conjugate can be administered when necessary. Monitoring can be performed by the following technique: serum from a patient is tested in vitro for levels of soluble XNA antibodies (primarily of the G and M classes). The serum is first fractionated into individual immunoglobulin classes. These are then administered to separate microtitre plate wells coated with Galα1,3Galβ1,4GlcNAc-HSA. Bound antibody is detected with reporter enzyme conjugated anti-human IgG or -IgM antibody.

The present invention can also be used to provide an immunoadsorbent which is located outside of a subject's body and which acts to remove xenogenic natural antibodies of the subject which are involved in rejection of xenografts. Desirably the immunoadsorbent will be immobilised - e.g. it will be present in a column. Thus, xenogenic natural antibodies can be removed from blood or at least their titre can be reduced. This is advantageous since a transplant patient's own blood can be treated using this technique and then returned to the patient. Another benefit of this technique is in providing stocks of blood from blood donors, which can be packaged for later use and used in the treatment of transplant patients. Such treated blood is within the scope of the present invention.

The present invention also provides an apparatus in which at least one conjugate of the present invention is located in a chamber provided with an inlet and an outlet. Blood can be treated by passing it through this chamber via the inlet and outlet.

Preferably the protein component of the conjugate is a human protein. The term "human protein" is used here to include proteins which occur naturally in humans, as well as such proteins or derivatives thereof having the same or a substantially similar biological activity. Such proteins may be produced naturally or may be produced synthetically (e.g. by chemical synthesis or by using recombinant DNA technology).

Desirably the protein is a component of blood. For example, it may be serum albumin (which may be in natural or recombinant form) or another generally inert native or recombinant protein, which is non-antigenic and non-immunogenic in the un-conjugated state and which is not a ligand for a human receptor molecule.

Preferably the protein is soluble (rather than membrane bound).

The protein may be associated with a plurality of epitopes to provide a conjugate for use in the present invention by any appropriate technique. Preferably the epitopes are covalently bound to the protein. This may be done using a spacer molecule.

A reaction which can be used to introduce a three atom spacer is a Michael addition between an acroylylate derivative of a sugar and a protein, e.g. HSA (Roy *et al, J Chem Soc Chem Commun* 1709-1711 (1990)). The reaction consists of three main steps:
i. Amination of a reducing sugar (e.g. B-trisaccharide) :- incubation in saturated ammonium hydrogen carbonate for 3 days at 37°C.
ii. Acryloylation:- use acryloyl chloride in the presence of Na₂CO₃, in a methanol-water solvent.
iii. After extensive lyophilisation, and purification of the derivatised sugar by gel filtration and reversed phase-HPLC it is coupled to HSA in 0.1M Na₂CO₃, pH 10.0 for 2 days at 37°C. The product of the reaction is sugar-NH-CO-CH2-CH2-NH-HSA.

### (The same basic reaction can be used to couple a sugar to polyacrylamide.)

Alternatively a sugar with a two carbon allyl linker in place can be coupled to a protein by a method using reductive ozonolysis followed by reductive amination with sodium cyanoborohydride (Bernstein, M.A. and Laurance, D.H., *Carbohydr*. *Res*. **78** (1980)).

The epitopes will desirably include as a minimum structure the smallest ligand necessary for binding of XNAs, which is galactose linked in an alpha-configuration. Thus the epitopes are preferably saccharides and desirably are oligosaccharides, or mimics of these saccharide structures.

The epitopes are desirably saccharides and are preferably oligosaccharides. Mimics of any such saccharides or oligosaccharides are included within the scope of the present invention. The term "mimic" refers to any structure which acts in substantially the same manner as a saccharide or oligosaccharide in binding to an antibody.

Such mimics are known in the art (and are sometimes referred to as mimetics). For example, Shikhman A.R. and Cunningham M.W., (*J. Immunol*. **152**(9):4375 (1994)) describe the use of cytokeratin peptides which act as mimics of sugar epitopes by blocking anti-GlcNAc antibodies; Vaughan *et al* (*Xenotransplantation* **3**:18-23 (1996)) describe a synthetic octapeptide (DAHWESWL) which mimics the epitope Galα(1,3)Gal; and Koogman *et al* describe a different peptide (SSLRGF) which also mimics the epitope Galα1,3Gal.

The epitopes may include sulphate groups, sialic acid and α-galactose (or mimics thereof).

The conjugate may be a neoglycoprotein (a non-glycosylated protein to which saccharides of a defined structure have been attached). Neoglycoproteins are discussed by Adler *et al* (*J Biol Chem*, **270:** 5164-5171 (1995)) and have been used for probing carbohydrate-protein interactions. They can be produced by enzyme catalysed reactions (e.g. using glycosyltransferases or glycosidases) or by chemical synthesis techniques. The methodology for chemical glycosylation of human serum albumin (HSA) and bovine serum albumin (BSA) is well established, as is the chemical synthesis of B-trisaccharide (Jacquinet *et al*, *JCS Perkin T:* 326-330 (1981)). Indeed a series of suitable Galα1,3Gal based neoglycoproteins are available from Dextra Laboratories Ltd, Reading, UK. This company produces HSA-conjugated Galα1,3Gal and HSA conjugated-Galα1,3Galβ1,4GlcNAc. Both of these can be obtained as either the 3- or 14- atom spacer molecules. Similarly the company produces four 3SA-based neo-glycoproteins (HSA and BSA refer to human serum albumin and bovine serum albumin respectively).

Where it is desired to use xenotransplants from pigs, preferred epitopes for use in producing the conjugate include two galactose moieties. Desirably the two galactose moieties are linked by an α1,3 linkage. Thus Galα1,3Gal is a favoured epitope for use in the present invention. This epitope may be present as part of a larger oligosaccharide structure. For example, it may be part of a trisaccharide: e.g. Galα1,3Galβ1,4GlcNAc. Other structures which can be used include: -
1) Multi-antennary oligosaccharides which may present up to 4 Galα1,3Gal termini. These oligosaccharides can be readily purified from natural sources e.g. bovine or porcine thyroglobulin.
2) Glycolipid derived α-galactosylated oligosaccharides.
3) Synthetic glycosphingolipids bearing the correct terminal structure. Thus creating a neosphingoglycoprotein.

The disaccharide Galα1,3Gal may itself be used.

As alternatives to the epitopes discussed above, other epitopes could be used. These include: oligosaccharides with terminal galactose α-linked to a subterminal residue which need not be galactose. The α-linkage need not be a 1,3 linkage Examples are: melibiose: Galα1,6Glc; raffinose : Galα1,6(Fuc α1,4) Glc and arabinogalactan.

Where xenografts are used from animals other than pigs then appropriate epitopes in respect of those animals can be used to prepare a conjugate of the present invention. In any event, Galα1,3Gal is expressed widely among non-primate mammals and New World Monkeys and is likely to be a major epitope in xenotransplantation of organs from a wide variety of mammals. However, as in the case of the pig, other epitopes may be used. Furthermore other species may present unique epitopes which will react with human XNAs. As with Galα1,3Gal none of these epitopes are likely to be expressed by the adult human.

Various preferred epitopes which can be used in the present invention are the carbohydrates given in the list below. (However other epitopes can of course be used (e.g. the xenoantigens described in W093/03735).)

### List of preferred epitopic structures

(i) Any carbohydrate terminating in Galα1,3Gal (as previously claimed) including blood group B-related oligosaccharides, the pentasaccharide Galα1, 3Galβ1 , 4GlcNAcβ1, 3Galβ1, 4Glc, Galα1,3Galβ1,3GalNAcβ1,4Galβ1,4Glc and blood group I-accive carbohydrates (Dabrowski, U., *et al*, *J. Biol*. *Chem.* **259**:7648-7651 (1984)).
(ii) n-glycolylneuraminic acid. This is a monosaccharide expressed in pig tissue but not on normal human tissue; however it has been described on human tumours (Fukui, Y., *et al*, *Biochem. Biophys*. *Res. Commun*. **160**:1149-1154 (1989)). In these respects n-glycolylneuraminic acid is similar to Galα1,3Gal.
(iii) Galα1,XGal where X could be 2, 4, or 6. There is some evidence that some XNAs recognise these structures (Wieslander, J., *et al*, *Glycoconjugate J.* **7**:85-100 (1990)).
(iv) Blood group A or A-like substances. There is evidence that these structures are expressed on pig heart (Cooper, D., *et al, Transpl*. *Immunol.* **1**:198 (1993)) and kidney (Jalali-Araghi, K., and Macher, B.A., *Glycoconjugate J*. **11**:266-271 (1994)). Experimental evidence is presented in Table 1 herein that anti-A antibodies are elicited in primate recipients of kidney xenografts:
   Infusion of A and B carbohydrates has been performed in the past to control pathologies resulting from blood group incompatibility (Romano, E.L., *et al*, *Transplantation Proceedings* **19**:4475-4478 (1987)).
(v) T- and T-related antigens. Humans have significant titres of antibody against the T-antigen. (Thomsen Freidenreich antigen = Galβ1,3GalNAc-). Although this does not normally lead to problems as the disaccharide - at least in pig and human lymphocyte membranes - is cryptic, that is normally capped by non-immunogenic neuraminic acid (Newman *et al*, *Eur. J. Biochem.* **64**:373-380 (1976)). It is possible that this capping is not complete in all tissues carried in the porcine xenograft. Furthermore, genetic manipulation of xenografts e.g. the ablation of terminal Galα1,3Gal may expose T-antigen.
   Humans also carry natural antibodies against the Tn antigen (GalNAc-O-Ser/Thr) a normally cryptic antigen which can be generated by failure of the tissue to add an overlying galactose (Thurnher *et al, J. Clin*. *Invest.* **91:**2103-2110 (1993)).
   The T-antigen is likely to be a common substituent of mucin (O-linked) oligosaccharides. To test for anti-T antibodies in the serum of primate recipients of pig cardiac xenografts.
   IgG and IgM subclasses were incubated with fixed endothelial cells in the presence of high molecular weight pig stomach mucin treated to remove α-galactosyl groups and neuraminic acid (Table 2). The removal of α-galactose by coffee bean α-galactosidase was confirmed by IB-4 lectin binding studies (date not shown). In a preliminary experiment we found that the residual binding activity, probably due to T-antigen recognition, consistently accounted for -15-20% of the antibody binding to fixed endothelial cells and was confined to the IgM class (unlike anti-Galα1,3Gal, which can be of either IgG or IgM class).
(vi) poly-N-acetyllactosamine and lactosamine (-Galβ1,XGlcNAc-)n where n=1 or >1 and X may be 3 or 4. Although these structures are common modifications of human oligosaccharides, modified versions with A- B- and B-like termini may be particular to pigs. Not only will these oligosaccharides bind natural antibodies, they may be a target for lactosamine binding lactins (LBLs, (Feizi *et al*, *Biochemistry* **33**:6342-6349 (1994)) which could attract inflammatory cells to the xenograft.
(vii) 3-sulphated galactose (SO₄-3Galβ-). This has been previously described as a potential xenoantigen (Samuelsson and Cairns, in Complex Carbohydrates in Drug Research, Alfred Benzon Symposium **36**, 368-379. ed. Bock, Clausen (1994)) since it is expressed in normal human tissues as a cryptic antigen.
(viii) peptide epitopes which compete with Galα1,3Gal for binding to xenogeneic natural antibodies. These may be peptide sequences determined by phage display techniques or naturally occurring sequences. An example is a sequence or sequences on cat skeletal muscle superfast myosin which can bind a monoclonal anti-galα1,3Gal monoclonal antibody (Kirkeby, S., *Cell Tissue Res.* **283**:85-92 (1996)).
(ix) N-acetyl-β-D-glucosamine (GlcNAc). Cooper has identified anti-GlcNAc specificity among human antibodies eluted from pig hearts (Cooper, D., *et al*, *Transpl. Immunol.* **1**:198 (1993)). however it is well known that human serum contains a natural antibody against GlcNAc (Emmrich *et al*, *J. Exp. Med* **161:**547 (1985)) which may be cross-reactive with keratin (Shikhman and Cunningham, 1994). In the latter paper it was commented that this anti-carbohydrate antibody only bound *BSA-conjugates* carrying a minimum of 20 GlcNAc residues. Albumin has a distinct advantage over many other potential "scaffolds" in that it is big enough to carry a large number of epitopes, and will still be soluble.

In a preferred embodiment of the present invention the conjugate comprises an epitope capable of being bound by a xenogenic natural antibody as well as a moiety which binds to liver hepatocytes (desirably with high specificity). This moiety preferably comprises a terminal β-linked galactose since this binds to a hepatic protein/asialoglycoprotein receptor found on hepatocytes (see Virgolini *et al*, *Nucl*. *Med*. *Commun.* **12**:507-517 (1991)). The conjugate, together with xenogenic natural antibodies to which it is bound, can be cleared from circulation via the liver in a rapid manner.

The conjugate of the present invention may be provided in a kit, optionally including instructions for its use in preventing or alleviating rejection.

It will normally be provided in sterile form as a pharmaceutically acceptable composition, which may include additional ingredients, e.g. buffers, excipients, etc.

The conjugate may be provided in sterile saline or saline - dextrose, for example.

For ease of administration, it may be provided in unit dosage form. It may be in a form adapted for injection or infusion into a patient.

The conjugate can be used for the manufacture of a medicament for use in preventing the rejection of a xenograft (e.g. a discordant xenograft) or at least in reducing the rate or extent of rejection. Desirably the medicament is used for preventing rejection of a xenograft from a human recipient.

It should be noted that although the conjugates of the present invention has been discussed above mainly in relation to xenotransplantation, they have other potential applications. For example, they may be useful in treating diseases in which xenoantigens (e.g. Galα1,3Gal) are implicated. The term "treating" is used herein to encompass prophylactic treatment as well as treatment of patients already having a particular disease.

Several human diseases involve recognition of Galα1,3Gal by anti-Galα1,3Gal antibody e.g. Chagas disease and Leishmania (Avila *et al*., *J. Immunol.* **142**:2828-2834 (1989)) and ideopathic myelofibrosis (Leoni *et al., British J. Haematology* **85:**313-319 (1993)). Towbin et *al., J. Exp. Med.* **166**:419-432 (1987) report that a natural protein-linked conjugate Galα1,3Gal - mouse laminin is a very good inhibitor of the anti-Galα1,3Gal antibody elicited in Leishmaneisis sera and also normal human sera. Thus the present invention is of potential utility in treating Chagas disease, Leishmania and ideopathic myelofibrosis.

The present invention will now be described by way of example only, with reference to the accompanying tables and drawings; wherein:
- TABLE 1: shows the agglutination of human A+ and B+ erythrocytes (0.3%) by whole IgM fractions of pre- and post Tx sera.
Serum was collected from primate recipients of either pig cardiac xenografts (W544, W141, and W135) or pig kidney xenografts (T381 and V337). This was size fractionated to generate pools containing predominantly IgG or IgM. The IgM fractions were pre-adsorbed with human O erythrocytes to eliminate reactivity towards epitopes other than A- or B-substance. The IgM was then mixed with human A1 or B erythrocytes to a final dilution of 1:40 with respect to the original serum. Figures in parentheses indicate the number of days elapsed between surgery and collection of serum. "Pre-" denotes that the serum was collected before the xenografting operation. After 2 hours at room temperature the erythrocytes were inspected for signs of agglutination. Values in the 2nd and 3rd columns represent the lowest dilution of IgM (with respect of neat serum) that is able to cause complete agglutination of the erythrocytes. As controls, human A1 and B erythrocytes were also challenged with monoclonal anti-A or B group antibodies.
- TABLE 2: shows the inhibition of antibody binding to fixed porcine aortic endothelial cells with agalactosyl-, asialo-high molecular weight fraction of pig stomach mucin.
IgG and IgM fractions from serum samples were mixed with either HSA or desialylated, α-galactosidase-treated pig stomach mucin (high mol. weight fraction) so that the final dilution of antibody with respect to serum was 1:12 and the final concentration of inhibitor was 100µg/ml. 'W544' and 'W141' are two cynomolgus monkey recipients of hDAF-expressing pig heart xenografts. Numbers represent days post transplant. 'Pre' denotes a pre-transplant sample. After 2 hours at 4°C the mixture were applied to wells coated with fixed porcine aortic endothelial cells. After a 2 hour incubation period at room temperature, bound antibody was detected with peroxidase-conjugated anti-human IgM or IgG. Table 2 indicates the percentage change in binding of immunoglobulin in the presence of the mucin as opposed to HSA.

### Inhibition of binding of XNA IgM to B-trisaccharide-HSA by soluble sugars and glycoconjugates.

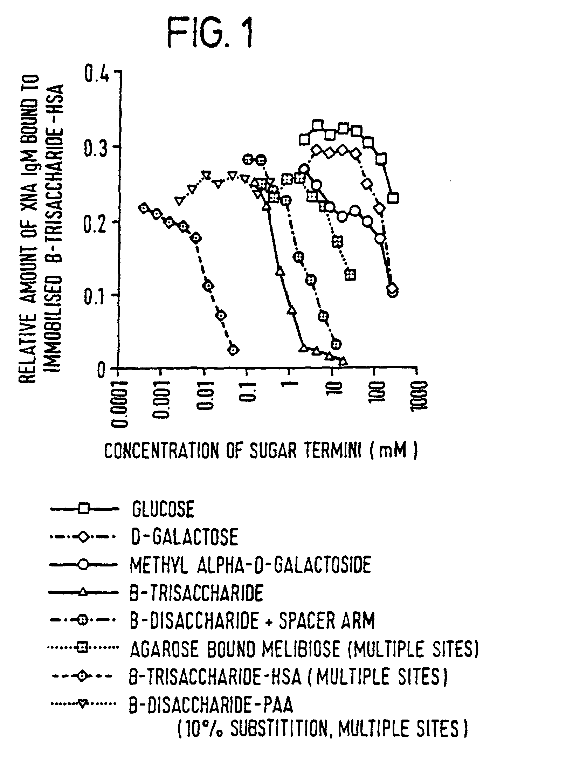

- FIGURE 1: shows the results of a competitive binding inhibition assay with conjugates which can be used in accordance with the present invention, as well as the results of this assay using free oligosaccharides.
This figure indicates that, mole for mole, B-trisaccharide-HSA is approximately 2 orders of magnitude better at binding XNA IgM than free B-trisaccharide even when the total number of epitopes presented is taken into account. One hypothesis is that the arrangement of epitopes on the protein enables several arms of the immunoglobulin to be bound at once, increasing the affinity of the immunoglobulin. Furthermore the protein scaffold may inhibit the free motion of the carbohydrate epitopes, increasing their antigenicity. These properties are not automatically shared by all conjugates (such as those mentioned in W093/03735) and are all significant advantages of using a protein backbone.

### A two stage chromium release assay on normal porcine endothelial cells (PAE 68#3) involving an initial incubation with serial dilutions of affinity isolated IgM or IgG followed by 1/8 baby rabbit complement.

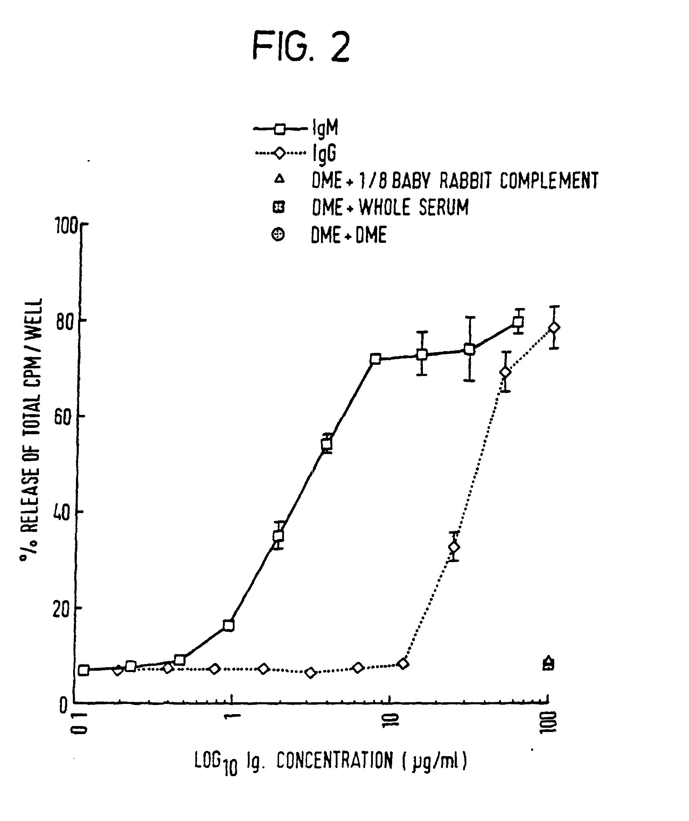

- FIGURE 2: shows the results of a two stage chromium release assay on normal porcine aortic endothelial cells (designated here as PAE-68#3) involving an initial incubation with serial dilutions of affinity-isolated IgM or IgG followed by 1/8 baby rabbit complement.
The amount of chromium released reflects the extent of complement-mediated lysis initiated by XNA binding.

### A two stage chromium release assay on normal porcine endothelial cells (PAE-68#4) involving an initial incubation with either human affinity-isolated IgM (10µ/ml) in the presence of four different sugars followed by 1/8 baby rabbit complement.

- FIGURE 3: shows the results of a two stage chromium release assay on normal porcine endothelial cells (PAE-68#4) involving an initial incubation with either human affinity-isolated IgM (10µg/ml) or IgG (80µg/ml) in the presence of three different sugars or HSA-B-trisaccharide glycoconjugate followed by 1/8 baby rabbit complement

### Inhibition of binding of IgG or IgM from pooled human serum to fixed PAEC by pre-incubation with B-trisaccharide-HSA (HSA-3)

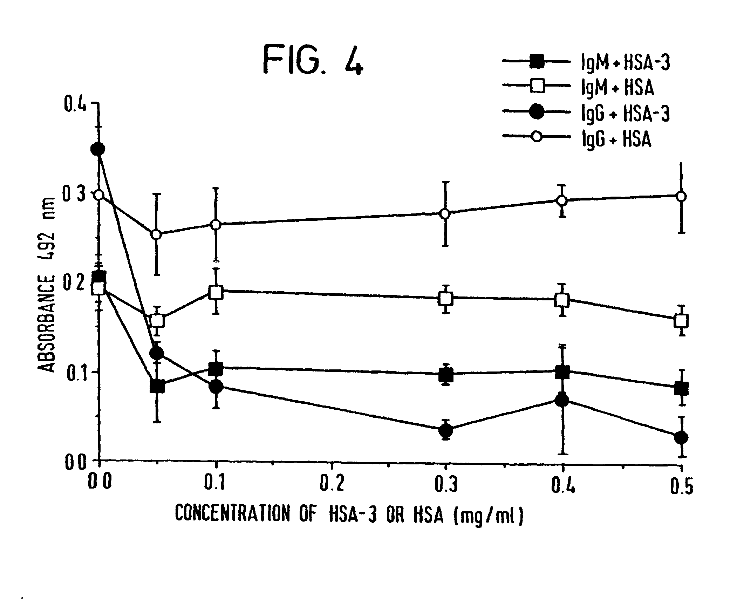

- FIGURE 4: shows the results of a competitive binding inhibition assay in which total IgM and IgG fractions of human serum were mixed with varying concentrations of B-trisaccharide-HSA before incubating with fixed monolayers of cultured porcine aortic endothelial cells in wells of a microtitre plate.
In a second step, 1/200 peroxidase conjugated anti-human IgM and IgG antibody (Sigma) is added. Bound peroxidase is measured (vertical axis) and is related in a simple fashion to the amount of serum antibody attached to the porcine cells. The drop in immunoglobulin binding in the presence of the conjugate signifies the importance of Galα1,3Gal in the binding of pre-transplant IgM and IgG to porcine tissue.
Note that most of the binding of both IgG and IgM is through Galα1,3Gal recognition but the efficacy of the conjugate is superior against IgG. It may be the case that only partial inhibition of binding is sufficient to substantially reduce the cytotoxic effects of the antibodies.

### w544: post Tx AAG IgG

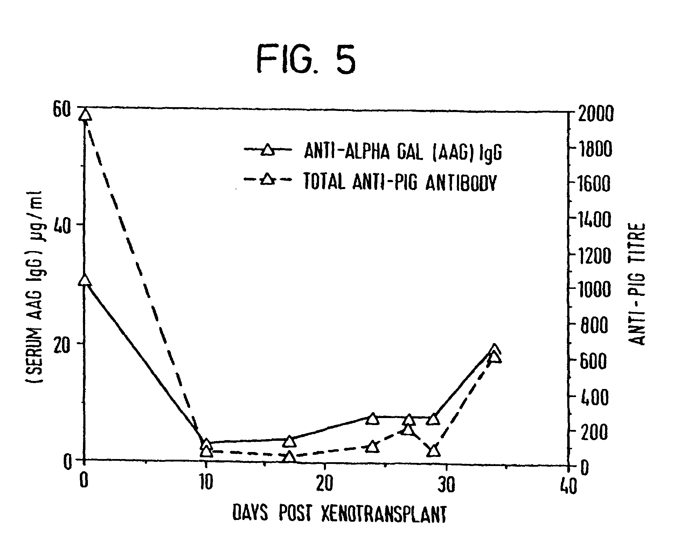

- FIGURE 5: shows the changes in anti-αGal antibodies in the serum of a primate (w544, which is a cynomolgus monkey which had received a transgenic (human DAF expressing) pig heart xenograft and which was also on a low cyclophosphamide (immunosuppressant) regime) following grafting of a pig heart (genetically modified to be overcome hyperacute rejection).
IgM and IgG were initially separated by gel filtration chromatography and the anti-αGal component in the IgG pool was measured by an ELISA method using B-trisaccharide-HSA as a target. The histogram shows an increase in anti-αGal IgG concentration in the recipient's serum shortly before rejection of the xenograft. Also shown is the titre of generalised anti-pig antibody as measured by a complement-dependent pig red blood cell lysis assay.

### Inhibition of binding of post-xenotransplant IgG to PAEC in the presence of H-trisaccharide-HSA

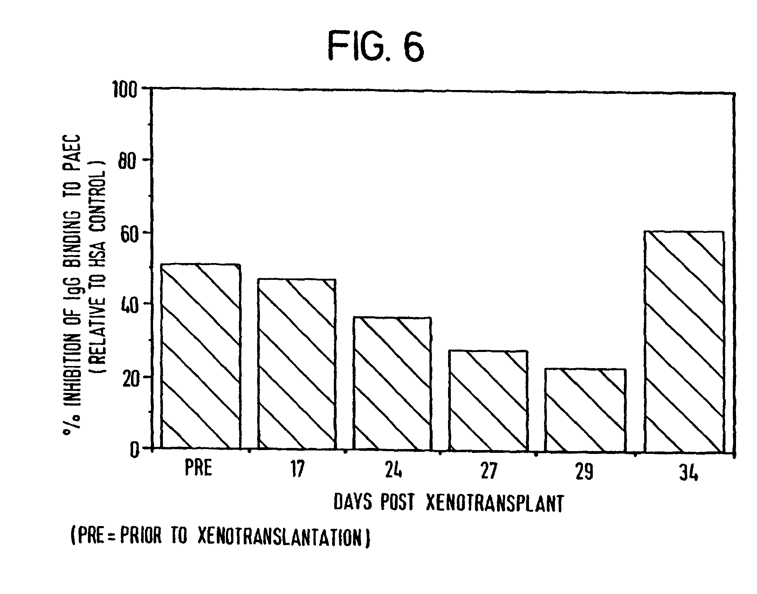

- FIGURE 6: indicates how important recognition of Galα1,3Gal is in the binding of recipient post-xenotransplant immunoglobulin to porcine cells.
Post-xenograft serum from the recipient described in Figure 5 was applied to fixed aortic endothelial cell lines in the presence of either HSA or B-trisaccharide-HSA (0.2mg/ml). B-trisaccharide-HSA intervention had its greatest effect against pre-xenotransplant IgG and IgG rom serum collected just before rejection. Thus, despite the continual presence of anti-αGal antibody as measured by ELISA, the competitive strength of the conjugate in the presence of pig cells was variable, and probably related to affinity changes in the elicited antibody. It is encouraging that the anti-αGal IgG peak chat precedes rejection in this instance is relatively sensitive to competition with the conjugate.

### Relative staining of PAEC by affinity-purified IgM

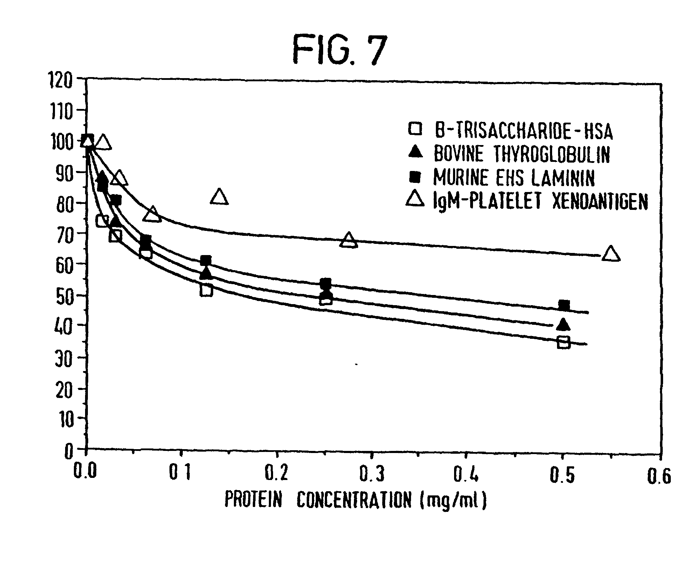

- FIGURE 7: shows a comparison of the inhibitory potency of B-trisaccharide-HSA, other naturally α-galactosylated glycoproteins and XNA IgM target xenogantigens.
Affinity purified anti-Galα1,3Gal IgM from human serum was applied to fixed porcine aortic endothelial cells (PAEC) after pre-incubation with either HSA, B-trisaccharide-HSA, murine EHS laminin, bovine thyroglobulin, or porcine platelet xenoantigens. The horizontal axis represents the amount of XNA IgM bound to the PAEC which is detected with horseradish peroxidase conjugated human anti-IgM antibody.

### Example

XNA were purified from 500 ml human serum by affinity chromatography on thyroglobulin-Sepharose. Thyroglobulin is a glycoprotein rich in α-galactose carbohydrate. IgG and IgM subclasses were separated in a second step by gel filtration (this procedure fractionates on the basis of size).

A selection of free and conjugated sugars was tested for ability to inhibit the binding of the XNA IgM to an α1,3-galactosylated glycoprotein (referred to herein as "B-trisaccharide-HSA". This is a construct comprising a plurality of Galα1,3Galβ1,4GlcNAc moieties (i.e. B-trisaccharides) linked to human serum albumin through three atom spacers, and is available from Dextra Laboratories Ltd, Reading, UK, under product number NGP2334) (Figure 1). The most striking result was that B-trisaccharide-HSA was a hundred-fold more inhibitory towards IgM binding than unconjugated Galα1,3Galβ1,4GlcNAc (referred to in Figures 1 and 3 as "B-trisaccharide"). These figures also refer to "B-disaccharide". This is Galα1,3Gal. In some cases this is used with a spacer arm. The spacer arm is OCH₂CH₂CH₂NH₂. Figure 1 refers to B-disaccharide-PAA. This is a conjugate of polyacrylamide and a plurality of Galα1,3Gal epitopes and polyacrylamide, as described in Rieben *et al*, *Xenotransplantation* **2** 98-106 (1995). Each disaccharide epitope is linked to a CONH moiety on the polyacrylamide through a spacer of the composition CH₂CH₂CH₂O. It is obtainable from syntesome GmbH and was used for comparison with B-trisaccharide-HSA. A 10% mol substitution form was used.

The ability of affinity-isolated human IgG and IgM to trigger lysis of porcine endothelial cells was determined by a two-stage ⁵¹chromium release assay. Briefly normal porcine endothelial cells were grown to confluence in 96-well plates before being washed and labelled with ⁵¹chromium. Excess label was removed and the cells were exposed to different concentrations of XNA. Unbound antibody was removed and 1/8 baby rabbit complement added for 1 hour at 37°C. Cell lysis was expressed as a percentage of the total counts associated with the endothelial cells, which could be detected in the medium after incubation of the cells with complement.

Both immunoglobulins bound to the endothelial cells resulting in cell lysis through their fixation and subsequent activation of the baby rabbit complement. In control experiments antibody or baby rabbit complement alone resulted in no specific cell lysis (Figure 2). The IgG fraction diluted out to 12.5µg/ml whereas the value for IgM was 0.47µg/ml. There was an approximate ten-fold difference in the values for 50% lysis, c.32µg/ml for IgG compared to 3.2µg/ml for IgM.

Antibody binding (and hence lysis) to the porcine endothelial cells was then inhibited with various sugars. Affinity-isolated IgM (10µg/ml) or IgG (80µg/ml) was incubated with endothelial cells as before in the presence of varying concentrations of four different sugar preparations. The cells were then washed and exposed to baby rabbit complement as previously described.

The most potent inhibitor of cell lysis and therefore antibody binding in the presence of either immunoglobulin was the B-trisaccharide-HSA conjugate (Figure 3). As an inhibitor of XNA IgM binding this was over ten-fold more effective when compared to the unconjugated B-trisaccharide which in turn was superior to B-disaccharide (i.e. Galα1,3Gal) in the presence of IgM. The effect was less marked for IgG but still significant. There was minimal difference between B-trisaccharide and B-disaccharide for IgG binding, however they were more effective against IgG as opposed to IgM in their inhibitory action. Glucose, employed as a non-specific sugar, had no inhibitory action on either IgG or IgM binding even at a concentration of 10 mM.

The B-trisaccharide-HSA conjugate used was a very efficient inhibitor of the destruction of porcine cells elicited by the application of human XNA *in vitro.*

This compound could prolong the survival of pig-human xenotransplants, and since fewer molecules are likely to be required to give protection (compared to free α-galactosylated sugars), osmotic disturbances in the recipient may be diminished.

Further data supporting the present invention is provided in Tables 1 and 2 and in Figures 4 to 7. This data is discussed at pages 16 to 21.

### List of Abbreviations Used

BSA: bovine serum albumin
HSA: human serum albumin
Glc: glucose
GlcNAc: N-acetylglucosamine
GalNAc: N-acetylgalactosamine
Gal: galactose
XNA: xenogenic natural antibodies
IgG: immunoglobulin of the G class
IgM: immunoglobulin of the M class
B trisaccharide-HSA: A plurality of Galα1,3Galβ1,4GlcNAc epitopes conjugated to human serum albumin
DME: Dulbecco's Modified Eagle's Medium

**TABLE 1**

| antibody source | anti-A | anti-B |
|---|---|---|
| anti-A mAb | 1:800 | <1:50 |
| anti-B mAb | <1:50 | 1:1600 |
| 544 pre-Tx | <1:40 | <1:40 |
| w544 Tx + 17 days | <1:40 | <1:40 |
| w544 Tx + 24 days | <1:40 | <1:40 |
| w544 Tx + 27 days | <1:40 | <1:40 |
| w544 Tx + 34 days | <1:40 | <1:40 |
| w141 pre-Tx | <1:40 | <1:40 |
| w141 Tx + 21 days | <1:40 | <1:40 |
| w141 Tx + 29 days | <1:40 | <1:40 |
| w135 Pre-Tx | <1:40 | <1:40 |
| w135 Tx + 34 days | <1:40 | <1:40 |
| w135 Tx + 36 days | <1:40 | <1:40 |
| w135 Tx + 37 days | <1:40 | <1:40 |
| T381 pre-Tx | 1:320 | <1:40 |
| T381 + 2 days | <1:40 | <1:40 |
| T381 + 5 days | 1:320 | <1:40 |
| T381 + 8 days | 1:320 | <1:40 |
| V337 pre-Tx | <1:40 | <1:40 |
| V337 + 1 day | 1:320 | <1:40 |
| V337 + 4 days | <1:40 | <1:40 |
| V337 + 7 days | <1:40 | <1:40 |

**TABLE 2**

| sample | reduction in IgG binding (%) | reduction in IgM binding (%) |
|---|---|---|
| W544-pre | 14.5 | 17.4 |
| W544-13 days | 0 | 14.9 |
| W544-20 days | 0 | 12.5 |
| W141-4 days | 0 | 20.2 |

## Claims

1. A synthetic conjugate of a protein and a plurality of epitopes for use in medicine, wherein said epitopes are capable of binding to xenogenic natural antibodies.

2. A conjugate for use in medicine according to claim 1, wherein said protein does not cause an adverse immune response when present in humans.

3. A conjugate for use in medicine according to claim 1 or claim 2, wherein said protein is a human protein or a functional equivalent thereof.

4. A conjugate according to any preceding claim wherein said protein is a protein found in blood.

5. A conjugate for use in medicine according to any preceding claim, wherein said protein is serum albumin.

6. A conjugate for use in medicine according to any preceding claim wherein said epitopes are selected from an oligosaccharide or a mimic thereof, which includes a terminal galactose in an α conformation and which, optionally, is linked to the protein via a spacer molecule.

7. A conjugate for use in medicine according to any preceding claim having a plurality of epitopes which include α linked galactose.

8. A conjugate for use in medicine according to claim 7 having a plurality of epitopes which include Galα1,3Gal.

9. A conjugate for use in medicine according to any preceding claim which further comprises a moiety which binds to liver cells.

10. A conjugate for use in medicine according to claim 9 wherein the moiety which binds to liver cells comprises a β-linked galactose

11. A conjugate according to any of claims 1 to 10 for use in preventing rejection of a xenograft or at least of reducing the extent or rate of rejection.

12. A conjugate according to any of claims 1 to 10 for use in treating a disease in which an epitope capable of binding to a xenogenic natural antibody is implicated (e.g. Chagas disease, Leishmania or ideopathic myelofibrosis).

13. A conjugate according to any of claims 1 to 10 for use in treating blood removed from a blood donor to reduce the number of xenogenic natural antibodies present.

14. A conjugate according to claim 13 wherein the conjugate is immobilised.

15. Apparatus suitable for use in a method according to claim 13 or 14 including an immunoadsorbent comprising at least one conjugate as described in any of claims 1 to 10, a chamber in which that conjugate is retained and a fluid inlet and outlet.

16. Blood treated according to the method of claim 13 or claim 14.

17. A pharmaceutically acceptable composition comprising a conjugate as described in any of claims 1 to 10.

18. A pharmaceutically acceptable composition according to claim 17 adapted for use in injection or infusion.

19. A kit comprising a conjugate as described in any of claims 1 to 10, blood according to claim 16, a pharmaceutically acceptable composition according to claim 17 or 18, or an apparatus according to claim 15; including instructions for use:
a) in preventing rejection of xenografts or at least in reducing the rate or extent of rejection, or
b) in treating a disease in which an epitope capable of binding to a xenogenic natural antibody is implicated (e.g. Chagas disease, Leishmania or ideopathic myelofibrosis).

20. The use of a conjugate according to any of claims 1 to 10 in the manufacture of a medicament for preventing rejection of xenografts or at least for reducing the extent or rate of rejection.

21. The use of a conjugate according to any of claims 1 to 10 in the manufacture of a medicament for treating a disease in which an epitope capable of binding to xenogenic natural antibodies are bound (e.g. Chagas disease, Leishmania or ideopathic myelofibrosis).

## Patentansprüche

1. Synthetisches Konjugat eines Proteins und einer Vielzahl an Epitopen zur Verwendung in der Medizin, worin diese Epitope zur Bindung von xenogenen natürlichen Antikörpern fähig sind.

2. Konjugat zur Verwendung in der Medizin nach Anspruch 1, worin das Protein keine schädliche Immunreaktion hervorruft, wenn es im Menschen vorkommt.

3. Konjugat zur Verwendung in der Medizin nach Anspruch 1 oder Anspruch 2, worin das Protein ein Humanprotein oder ein funktionelles Äquivalent hiervon ist.

4. Konjugat nach einem der vorangehenden Ansprüche, worin das Protein ein Protein ist, das im Blut gefunden wird.

5. Konjugat zur Verwendung in der Medizin nach einem der vorangehenden Ansprüche, worin das Protein Serumalbumin ist.

6. Konjugat zur Verwendung in der Medizin nach einem der vorangehenden Ansprüche, worin die Epitope ausgewählt sind aus einem Oligosaccharid oder einem Imitat hiervon, das eine terminale Galactose in einer α-Konformation umfaßt und das wahlweise über ein Spacermolekül an das Protein gebunden ist.

7. Konjugat zur Verwendung in der Medizin nach einem der vorangehenden Ansprüche mit einer Vielzahl an Epitopen. die α-verknüpfte Galactose umfassen.

8. Konjugat zur Verwendung in der Medizin nach Anspruch 7 mit einer Vielzahl an Epitopen, die Galα1, 3 Gal umfassen.

9. Konjugat zur Verwendung in der Medizin nach einem der vorangehenden Ansprüche das ferner einen Rest umfaßt, der an Leberzellen bindet.

10. Konjugat zur Verwendung in der Medizin nach Anspruch 9, worin der Rest, der an Leberzellen bindet, eine β-verknüpfte Galactose umfaßt.

11. Konjugat nach einem der Anprüche 1 bis 10 zur Verwendung bei der Verhinderung der Abstoßung eines Xenotransplantats oder zumindest zur Verringerung des Ausmaßes oder der Geschwindigkeit der Abstoßung.

12. Konjuat nach einem der Ansprüche 1 bis 10 zur Verwendung bei der Behandlung einer Erkrankung, worin ein Epitop beteiligt ist das zur Bindung eines xenogenen natürlichen Antikörpers fähig ist (beispielsweise Chagas Erkrankung, Leishmaniose oder ideopathische Myelofibrose).

13. Konjugat nach einem der Ansprüche 1 bis 10 zur Verwendung bei der Behandlung von Blut, das von einem Blutspender stammt, um die Anzahl an vorhandenen xenogenen natürlichen Antikörpern zu verringern.

14. Konjugat nach Anspruch 13, worin das Konjugat immobilisiert ist.

15. Gerät, das zur Verwendung in einem Verfahren nach Anspruch 13 oder 14 geeignet ist, das eine Immunabsorptionseinheit aufweist, welche zumindest ein Konjugat nach einem der Ansprüche 1 bis 10, eine Kammer, in der das Konjugat zurückgehalten wird und einen Flüssigkeitseinlaß und -auslaß umfaßt.

16. Blut, das gemäß dem Verfahren nach Anspruch 13 oder Anspruch 14 behandelt wird.

17. Pharmazeutisch annehmbare Zusammensetzung, die ein Konjugat nach einem der Ansprüche 1 bis 10 umfaßt.

18. Pharmazeutisch annehmbare Zusammensetzung nach Anspruch 17, die zur Verwendung bei der Injektion oder Infusion angepaßt ist.

19. Kit. der ein Konjugat nach einem der Ansprüche 1 bis 10, Blut nach Anspruch 16, eine pharmazeutisch annehmbare Zusammensetzung nach Anspruch 17 oder 18 oder ein Gerät nach Anspruch 15 einschließlich Anleitungen zur Verwendung umfaßt
a) zur Verhinderung der Abstoßung von Xenotransplantaten oder zumindest Verringerung der Geschwindigkeit oder des Ausmaßes der Abstoßung, oder
b) zur Behandlung einer Erkrankung, worin ein Epitop beteiligt ist, das zur Bindung eines xenogenen natürlichen Antikörpers fähig ist (beispielsweise Chagas Erkrankung, Leishmaniose oder ideopathische Myelofibrose).

20. Verwendung eines Konjugats nach einem der Ansprüche 1 bis 10 zur Herstellung eines Arzneimittels zur Verhinderung der Abstoßung von Xenotransplantaten oder zumindest zur Verringerung des Ausmaßes oder der Geschwindigkeit der Abstoßung.

21. Verwendung eines Konjugats nach einem der Ansprüche 1 bis 10 zur Herstellung eines Arzneimittels zur Behandlung einer Erkrankung, worin ein Epitop gebunden ist, das zur Bindung an xenogene natürliche Antikörper fähig ist (beispielsweise Chagas Erkrankung, Leishmaniose oder ideopathische Myelofibrose).

## Revendications

1. Conjugué synthétique d'une protéine et d'une pluralité d'épitopes destiné à être utilisé en médecine, dans lequel lesdits épitopes sont capables de se lier avec des anticorps naturels xénogènes.

2. Conjugué destiné à être utilisé en médecine selon la revendication 1, dans lequel ladite protéine n'entraîne pas de réponse immunitaire indésirable lorsqu'elle est présente chez les humains.

3. Conjugué destiné à être utilisé en médecine selon la revendication 1 ou la revendication 2, dans lequel ladite protéine est une protéine humaine ou un équivalent fonctionnel de celle-ci.

4. Conjugué selon l'une quelconque des revendications précédentes, dans lequel ladite protéine est une protéine que l'on trouve dans le sang.

5. Conjugué destiné à être utilisé en médecine selon l'une quelconque des revendications précédentes, dans lequel ladite protéine est de la sérum-albumine.

6. Conjugué destiné à être utilisé en médecine selon l'une quelconque des revendications précédentes, dans lequel lesdits épitopes sont sélectionnés parmi un oligosaccharide ou une substance mimétique de celui-ci, qui comprend un galactose terminal selon une conformation α et qui, en option, est lié à la protéine via une molécule d'espacement.

7. Conjugué destiné à être utilisé en médecine selon l'une quelconque des revendications précédentes, présentant une pluralité d'épitopes qui comprennent un galactose lié selon α.

8. Conjugué destiné à être utilisé en médecine selon la revendication 7, présentant une pluralité d'épitopes qui comprennent Galα1,3Gal.

9. Conjugué destiné à être utilisé en médecine selon l'une quelconque des revendications précédentes, qui comprend en outre une fraction qui se lie aux cellules du foie.

10. Conjugué destiné à être utilisé en médecine selon la revendication 9, dans lequel la fraction qui se lie aux cellules du foie comprend un galactose lié selon β.

11. Conjugué selon l'une quelconque des revendications 1 à 10, destiné à être utilisé pour prévenir le rejet d'une xénogreffe ou au moins réduire l'étendue ou la vitesse de rejet.

12. Conjugué selon l'une quelconque des revendications 1 à 10 destiné à être utilisé pour traiter une maladie dans laquelle un épitope capable de se lier à un anticorps naturel xénogène est impliqué (maladie de Chagas, leishmaniose ou myélofibrose idiopathique).

13. Conjugué selon l'une quelconque des revendications 1 à 10, destiné à traiter du sang prélevé d'un donneur de sang pour réduire le nombre d'anticorps naturels xénogènes présents.

14. Conjugué selon la revendication 13, dans lequel le conjugué est immobilisé.

15. Appareil approprié pour être utilisé dans une méthode selon la revendication 13 ou 14, comprenant un immuno-adsorbant comportant au moins un conjugué tel que décrit dans l'une quelconque des revendications 1 à 10, une chambre dans laquelle ce conjugué est retenu et une entrée et une sortie de fluide.

16. Sang traité selon la méthode de la revendication 13 ou de la revendication 14.

17. Composition pharmaceutiquement acceptable comprenant un conjugué tel que décrit dans l'une quelconque des revendications 1 à 10.

18. Composition pharmaceutiquement acceptable selon la revendication 17, adaptée pour une utilisation en injection ou en perfusion.

19. Kit comprenant un conjugué tel que décrit dans l'une quelconque des revendications 1 à 10, du sang selon la revendication 16, une composition pharmaceutiquement acceptable selon la revendication 17 ou 18, ou un appareil selon la revendication 15 ; et comportant des instructions d'utilisation pour :
a) prévenir le rejet de xénogreffes ou au moins réduire la vitesse ou l'étendue du rejet, ou
b) traiter une maladie dans laquelle un épitope capable de se lier à un anticorps naturel xénogène est impliqué (par exemple la maladie de Chagas, la leishmaniose ou une myélofibrose idiopathique).

20. Utilisation d'un conjugué selon l'une quelconque des revendications 1 à 10, dans la fabrication d'un médicament destiné à prévenir le rejet de xénogreffes ou au moins réduire la vitesse ou l'étendue du rejet.

21. Utilisation d'un conjugué selon l'une quelconque des revendications 1 à 10 dans la fabrication d'un médicament destiné à traiter une maladie dans laquelle un épitope capable de se lier à des anticorps naturels xénogènes est impliqué (par exemple la maladie de Chagas, la leishmaniose ou une myélofibrose idiopathique).
